# EUROPEAN PATENT SPECIFICATION

(11) **EP 1 547 505 B1**
(45) Date of publication and mention of the grant of the patent: **09.12.2009**
(21) Application number: 03748143.9
(22) Date of filing: 11.09.2003
(51) Int. Cl.: A47K 5/12, A61L 9/12

(54) **MULTIFUNCTIONAL METERING DEVICE**
MULTIFUNKTIONALE DOSIERVORRICHTUNG
DOSEUR MULTIFONCTIONNEL

(30) Priority: 13.09.2002 ES 200202100
(43) Date of publication of application: 29.06.2005
(73) Proprietor: Tenko Ser 21 S.A., 48012 Bilbao (ES); Servicios de Contenedores Higienicos Sanitarios S.A., 48012 Bilbao (ES); Europea de Servicios e Higiene Euro-Servhi S.A., 48012 Bilbao (ES)
(72) Inventor: GONZALEZ DE ECHAVARRI, Victor, E-48011 Bilbao (ES)
(74) Representative: Gislon, Gabriele
(86) International application number: PCT/ES2003/000460
(87) International publication number: WO 2004/023960

(56) References cited:
- ES-U- 269 304
- ES-U- 1 044 608
- ES-U- 1 049 300
- US-A- 5 379 917

## Description

This invention relates to a new multipurpose dispenser, as indicated in the name and title of this specification, which has various functions and is not limited to dispensing pressurised or sprayed liquids, but which, at the same time or alternatively or simultaneously, according to its programming and requirements, can dispense room fresheners, insecticides, etc., by vaporisation, directly into the atmosphere. The multipurpose dispenser combines all these functions whereby it provides considerably important and advantageous characteristics with respect to the dispensing apparatuses and devices that are currently known for fulfilling similar purposes.

In addition, the multipurpose dispenser of the invention comprises a pressurising or spraying system which is controlled automatically to supply the product directly to the relevant channels, or into the atmosphere, and its purpose is to permit the cleaning, deodorisation, and the disinfection, sanitation or disinsectisation of sanitary equipment in the bathroom, toilet, public lavatory, etc, and also of the environments themselves, both public and private. This is achieved thanks to its automatic control, which enables the dispenser to operate according to the requirements and purpose of each place where it is installed, but which, in turn, offers the possibility of replacing the dropwise system directed at conduit channels, with a direct spraying action into the atmosphere.

Moreover, it comprises another system inside the same apparatus which, being controlled in the same automatic fashion but with a separate motor, is intended to direct an essence that can be either a deodorant, a room freshener or even an insecticide, directly into the atmosphere in the place where the apparatus is installed, which will mainly be a bathroom, as indicated above, but which can be any other place having other greater needs for refreshing, disinfecting or disinsecting the atmosphere.

All this is achieved with one single apparatus having the whole ensemble combined and automatically controlled, allowing said multiple functions to which the title of the invention refers, to exist either separately or in combination, and this consequently affords important advantages, both in terms of the manufacture and/or assembly, and the use of the actual dispensing apparatus.

### Background to the invention

A multi purpose dispenser for two products is known from US-A-5379917, wherein the one product is dispensed by pushing an actuating plate, where as the other product is dispensed by motor and a fan.

Known dispensers do not have all the functions and possibilities described in association with this multipurpose dispenser. As an example, Spanish Patent No. 9701087 and Spanish Utility Model No. 200101481 are known, which are merely liquid dispensing apparatuses. Also, Spanish Utility Model No. 99002960 is known which is only a room freshener device. The apparatuses in these documents are included among many others that are known conventionally in the market, all of them having limited possibilities, whereby they must be replaced depending on the intended purposes and the places where they are going to be installed. Contrary to this, the new multipurpose dispensing apparatus according to this invention can be used practically everywhere, and for practically all the currently known uses of bacteriostatics and room fresheners, and even insecticides.

In short, it is a combination of different elements in one and the same dispenser, which comprises two deposits holding different contents that can be used in combination, alternatively or totally independently, and which are controlled automatically by one single control that can be programmed according to the functions to be fulfilled. This combination and arrangement represents a great development in the state of the art, since a device having these characteristics is not known in the market, only those devices that are generally independent having one of the individual functions, not combined with the others.

### Description of the invention

Generally speaking, the multipurpose dispenser which is the subject-matter of this invention is intended to be able to dispense as much as may be required in order to obtain clean, disinfected, refreshed and sanitised places, by being able to avail of one or several of the functions as required each time, all this being possible by virtue of the combined dispensing of the essences and chemical liquids or products that already exist and are known in the market, mainly for bathrooms and toilets.

All this is achieved by providing a compact dispensing apparatus, having a unified, safe image owing to its restricted access in the form of a corresponding lock. The multipurpose dispenser provides some clear advantages, both in terms of its use, which we must remember will mainly be in bathrooms and public toilets, and in terms of its assembly, which is very simple owing to the fact that the dispenser is mounted on a base or frame which has a space prepared for installing each component element, and a front closing cover which in turn incorporates corresponding gratings for letting air or the dispensed product in and out. In turn, said cover makes it very easy to replace the deposits and batteries, without altering the operation of the apparatus in any way.

Therefore, the dispenser according to the invention can be described by its different parts, comprising a frame 1 which incorporates the device's component elements. The frame is already prepared to be installed preferably on the wall, by being screwed on at points 2, and it has a quadrangular shape being longer than wider, with its top face 3 arching outwards. In the lower part thereof it has an arrangement 4 for laterally housing the hinges for the front cover 5, which, once installed, will close off the dispenser by means of a corresponding safety lock indicated at 6.

In the central and right hand lower part thereof, the frame has a large cavity intended to receive a main deposit 7 of a product that is to be dispensed, either by a controlled dropwise action directed at the corresponding channels or pipes, or by being sprayed directly into the atmosphere. In turn, on the lower base there is an outlet 8 for the channels for the dispensed liquid, which will normally be small pipes, supported by a pin or support 9 provided on the rear wall of the frame.

In its lower left hand part thereof, the frame has a cavity that is intended to receive a smaller deposit 10 that preferably contains an "essence" to be dispensed by means of a cord 11. This cord 11 is incorporated in the small deposit 10 by means of a plug 12 designed especially for this purpose, and by means of an air current generated by a fan 13, via said cord 11, the said essence will be dispensed into the atmosphere so as to fulfil the function of room freshener, deodorant or even insecticide, if necessary.

It is evident that, in the top left hand part thereof, the frame has a small fan 13 that is driven automatically and conveniently by a motor 14 to generate an air current, which, in contact with the cord 11 of the essence deposit 10, will correctly dispense the part of the essence needed to purify or disinsect the atmosphere.

In the top central left hand part there is a second motor 15, separate from the motor 14 coupled to the fan 13, its output including a gear 16 coupled to other transmission gears 17 in order to transmit a movement to a pusher element 18 which acts on a push-button 19 associated with a valve 20 in the large deposit 7 containing one of the products to be dispensed.

In the top right hand part, the dispenser comprises a vertical standing plate 21 with an electronic control circuit that incorporates a control program for the whole automated dispenser. The program allows the device to be programmed as required. In the top right hand part, the dispenser has a cavity 22 for housing the electrical batteries for both the control system plate 21 and the motors, that is, motor 15 connected to gears 17 of pusher element 18 of push-button 19 of the large deposit 7, and motor 14 of fan 13.

The front cover 5 of the dispenser is curved outwards 23, and in the top left hand part thereof it is provided with a grating 24 which acts as outlet for the essence dispensed by the air current generated by fan 13. In turn, a central grating 25 acts as air passage for the current generated by the fan 13, and as it is located at the same height as the cord 11 in the essence deposit 10, it can also perform the function of room freshener when the fan is not is use, and this is because it is provided, in turn, with a grating 26 in the lower part thereof, which causes an air current to be generated inside the apparatus, with or without the fan. It should be pointed out that the air inlets and outlets can be inverted as required, as well as the rotation direction given to the fan, since it will often depend on where the dispensing apparatus is installed.

It should be noted here that the push-button 19 can act as a sprayer. In this case, the cover 5 includes a small window 27 to let the dispensed product contained in the deposit 7 exit to the outside.

As can be seen, one single dispenser presents a series of characteristics whereby a series of functions can be combined, and it can dispense disinfectants, cleaning agents, deodorants, room fresheners and insecticides, combining their functions, by providing all its elements in a simple but compact manner, which is safe both in terms of its manufacturing and its final use, even when replacing the deposits. The said combination can be multiplied, since on many occasions several dispensers will be installed, for example, in public places, and therefore the function of each dispenser can be selected in combination with the function of the others, and this provides an even better array of multiple functions which is the result of the advantages of this invention, and which are not known to exist in the market in one single apparatus of this kind.

### Brief description of the drawings

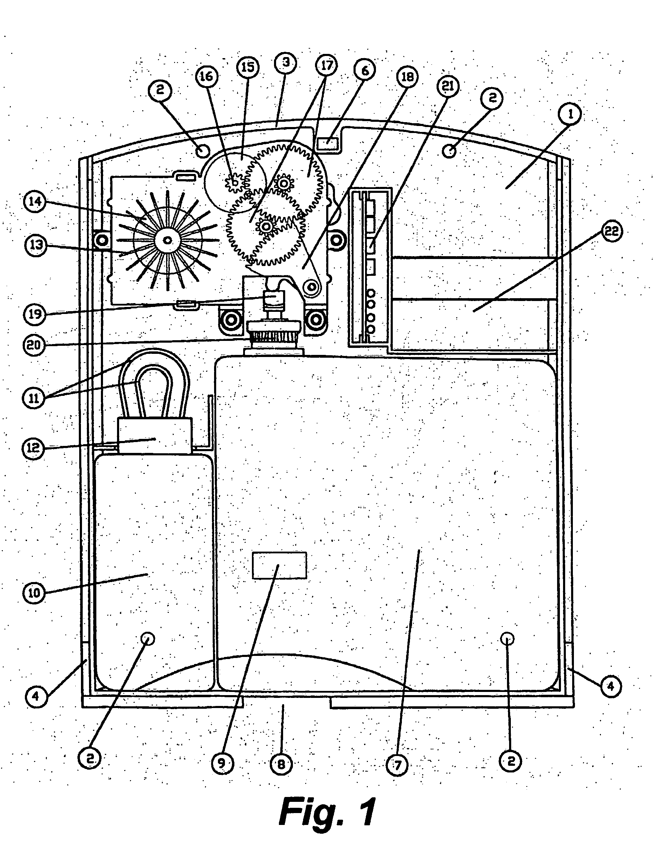

**Figure 1****.**- This is the main drawing and it represents a front view of the dispenser, without the front cover 5, in which the arrangement of all the main dispenser elements can be observed, beginning with the position in the central-right hand lower part thereof of the main deposit 7, which includes the valve 20 and the push-button 19. In the lower left hand part there is the smaller essence deposit 10, incorporating the cord 11. In the top left hand part, there appears the fan 13 which circulates the air treated with the product contents that is evaporated by the cord 11, and in turn the gears 16 and 17 which are driven by the motor 15, transmit the movement to the pusher element 18, which acts directly on push-button 19 of deposit 7. In the central right hand top part one can see where the dispenser control card or plate 21 is installed and to the right where the batteries 22 are located that power the control plate 21 and motors 15 and 14.

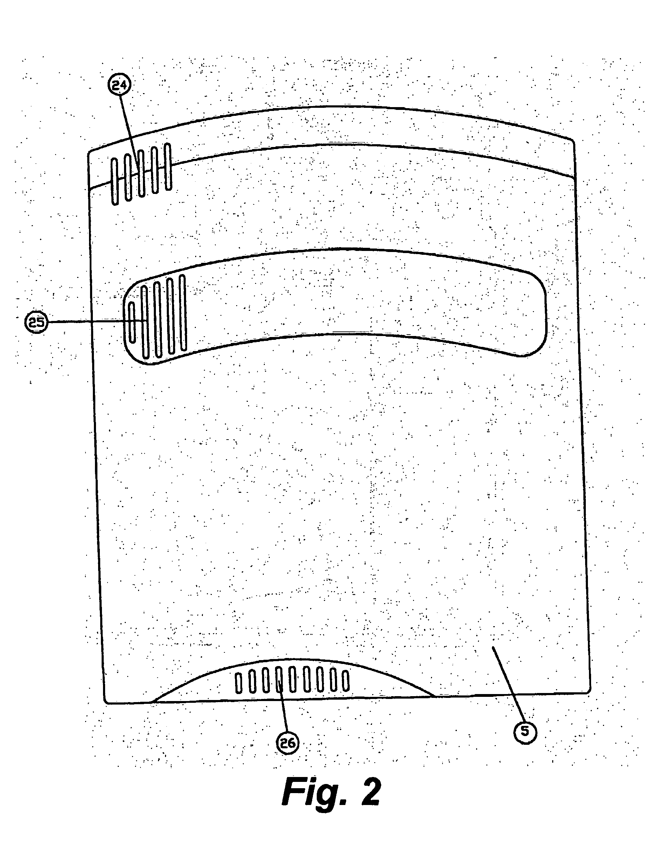

**Figure 2****.-** Represents the dispenser cover and the arrangement of the ventilation gratings 24, 25, and 26 that are placed at three different heights, and through which the air where the dispenser is installed will enter and then exit treated with the product that is evaporated by the cord 11. The air current will be generated mainly by the action of the fan 13 and the rotation direction given to the fan as required, which activates one or other of the dispenser gratings as an air inlet or outlet.

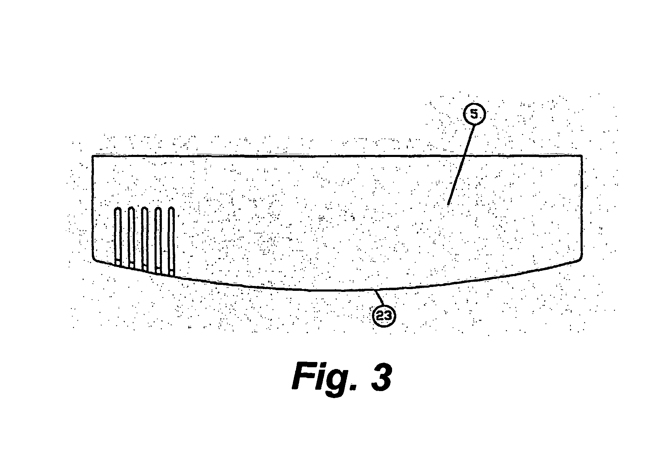

**Figure 3****.-** Represents the dispenser cover seen from above so as to reveal its front curved shape 23 whereby it can conceal all the dispenser devices, occupying as small a space as possible.

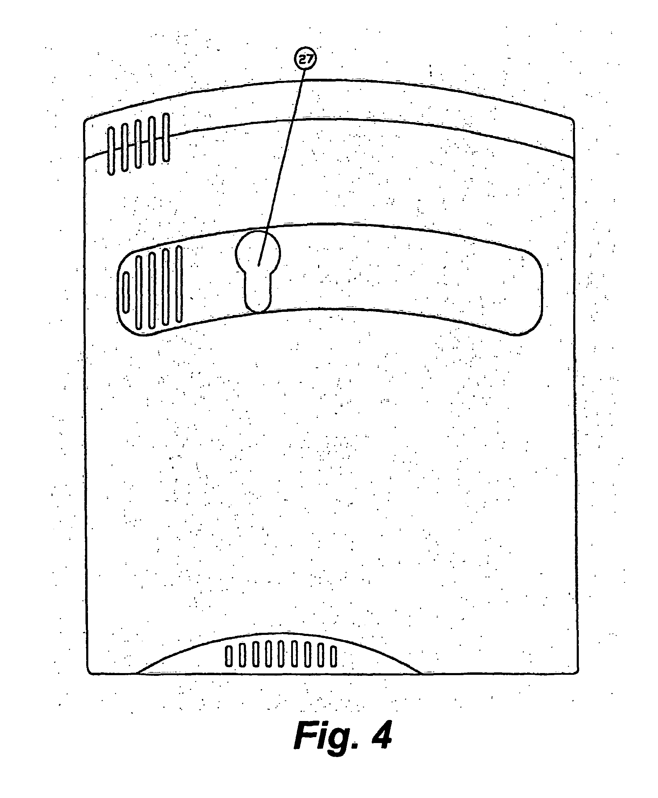

**Figure 4****.-** Represents an alternative cover that would be used in combination with the push-button 19 that can be used alternatively so that instead of directing the drops to certain channels or pipes, it could spray them directly into the atmosphere, in which case the dispenser cover would incorporate the window 27.

### Description of the preferred embodiment

With reference to the numbering used in the invention specification and in the accompanying drawings, the multipurpose dispenser is seen to comprise a frame 1 to be supported on the wall in a conventional manner, there being provided for this purpose the holes 2 in both the top and bottom thereof, with even a hole in the top central part thereof, said frame having an outlet 8 in the lower part thereof for the pipes and a pin 9 also for said pipes in the event that the product is dispensed dropwise to said channels or pipes from the push-button 19.

The larger size deposit 7 is arranged on the left hand central lower part of the frame, as illustrated in Figure 1. In the top left hand part thereof, the larger size deposit 7 includes a corresponding valve 20 activated by the push-button 19, which acts by directing the drops towards the channels or pipes or, alternatively, by spraying them directly outwards through the window 27 in the cover 5 (illustrated in Figure 4). The push-button 19 is activated by a pusher element 18, which is arranged in combination with gears 17 that receive the movement of a gear 16 connected to the output of a motor 15 powered by batteries housed at 22. The operation of the motor 15 is controlled by a control plate 21 on which all the dispenser functions are programmed, according to usage requirements.

The above arrangement is combined with that of the second deposit 10, located in the bottom left hand part. At the top thereof, the second deposit 10 comprises a cord 11 that evaporates the liquid contained in deposit 10. Above said cord there is a fan 13 that can generate an air current to dispense that evaporated by the cord 11. The fan 13 is driven by a motor 14 powered by the batteries housed at 22, and its operation is controlled according to that programmed on the control plate 21 whereby said plate controls both motors 14 and 15, combining the dispensing of each of the products contained in the deposits or containers 7 and 10, according to the requirements of each.

The whole dispenser mechanism described above is perfectly protected by a cover 5, which incorporates a safety lock 6 so that its handling is controlled by authorised personnel, when maintaining and replacing the deposits or containers 7 and 10 and the batteries 22 and even when programming the control plate 21. The function of the cover 5 is important, as can be seen, both in terms of manufacturing and use, and also because it provides the multipurpose dispenser with a unified and unique image.

## Claims

1. Multipurpose dispenser, of the type comprising a frame (1) provided with wall support means (2) and a front cover (5), at least one deposit for a product to be dispensed, and at least one dispensing mechanism activated by driving means powered by batteries, **characterised in that** it comprises two deposits (7) and (10) for products to be dispensed, where one of said deposits (7) incorporates a valve (20), which in the top part thereof has a push-button (19) that is activated by a pusher element (18) driven by the movement of a motor (15) that is transmitted via gears (16) and (17), said motor (15) being powered by said batteries located in a housing (22) and controlled by a control plate (21), which in turn controls and combines the operation of said motor (15) with that of another motor (14) that drives a fan (13) which circulates the air that is in contact with a product evaporated by a cord (11) located in the other of said deposits (10), all this being combined in a simultaneous or alternative fashion, according to the control plate (21) programming.

2. Multipurpose dispenser according to claim 1, **characterised in that** said cover (5) includes gratings (24, 25, 26) in the top, central and bottom parts thereof, respectively, which cause the air current generated by the fan (13) to circulate through them and the circulating air to come into contact with the product evaporated from deposit (10) by cord (11).

3. Multipurpose dispenser according to claim 2, **characterised in that** said push-button (19) is a sprayer push-button which dispenses the product contained in deposit (7) directly into the outside atmosphere via a window (27) included for this purpose in the front cover (5) of the dispenser, in simultaneous or alternative combination with the dispensing of the product contained in the deposit (10) by means of said gratings (24, 25 and 26).

4. Multipurpose dispenser according to claim 2, **characterised in that** said push-button (19) dispenses the product contained in deposit (7) into small pipes that are supported on the frame by a pin or support (9) and traversed by exit (8) and directed towards the corresponding channels or pipes, to be treated in a dropwise manner, in simultaneous or alternative combination with the dispensing of the product contained in the deposit (10) by means of said gratings (24, 25 and 26).

## Patentansprüche

1. Mehrzweckspender, der Art umfassend einen Rahmen (1), der mit Wandhalterungsmitteln (2) und einer vorderen Abdeckung (5) versehen ist, zumindest einem Behälter für ein zu spendendes Produkt, und zumindest einem Spendemechanismus, der durch Antriebsmittel aktiviert wird, die durch Batterien angetrieben werden, **dadurch gekennzeichnet, dass** er zwei Behälter (7) und (10) für zu spendende Produkte umfasst, wobei in einem der genannten Behälter (7) ein Ventil (20) eingebaut ist, welches im oberen Teil davon einen Druckknopf (19) hat, der durch ein Druckelement (18) aktiviert wird, das durch die Bewegung eines Motors (15) angetrieben wird, die über Zahnräder (16) und (17) übertragen wird, wobei der genannte Motor (15) durch die genannten Batterien angetrieben wird, die in einem Gehäuse (22) gelegen sind, und durch eine Steuerplatte (21) gesteuert wird, welche ihrerseits den Betrieb des genannten Motors (15) mit jenem eines anderen Motors (14) steuert und kombiniert, der einen Ventilator (13) antreibt, welcher die Luft in Umlauf bringt, die mit einem durch einen Docht (11) verdampften Produkt in Kontakt steht, die in dem anderen der genannten Behälter (10) gelegen ist, wobei all dies auf simultane oder alternative Art und Weise gemäß der Programmierung der Steuerplatte (21) kombiniert ist.

2. Mehrzweckspender nach Anspruch 1, **dadurch gekennzeichnet, dass** die genannte Abdeckung (5) jeweils Gitter (24, 25, 26) in den oberen, mittleren und unteren Teilen davon beinhaltet, welche bewirken, dass der durch den Ventilator (13) erzeugte Luftstrom durch dieselben zirkuliert, und die zirkulierende Luft mit dem aus dem Behälter (10) durch den Docht (11) verdampften Produkt in Kontakt gerät.

3. Mehrzweckspender nach Anspruch 2, **dadurch gekennzeichnet, dass** der genannte Druckknopf (19) ein Sprüh-Druckknopf ist, welcher das in dem Behälter (7) enthaltene Produkt direkt in die Außenatmosphäre über ein Fenster (27) spendet, das zu diesem Zweck in der vorderen Abdeckung (5) des Spenders beinhaltet ist, in simultaner oder alternativer Kombination mit dem Spenden des in dem Behälter (10) enthaltenen Produkts durch die genannten Gitter (24, 25 und 26).

4. Mehrzweckspender nach Anspruch 2, **dadurch gekennzeichnet, dass** der genannte Druckknopf (19) das in dem Behälter (7) enthaltene Produkt in kleine Rohre spendet, die auf dem Rahmen durch einen Stift oder eine Halterung (9) gehalten sind, und durch einen Ausgang (8) durchquert sind, und zu den entsprechenden Kanälen oder Rohren hin gerichtet sind, die tröpfchenweise zu behandeln sind, in simultaner oder alternativer Kombination mit dem Spenden des in dem Behälter (10) enthaltenen Produkts durch die genannten Gitter (24, 25 und 26).

## Revendications

1. Distributeur plurivalent du genre comportant un châssis (1) pourvu de moyens d'appui de paroi (2) et un couvercle avant (5), au moins un réservoir pour un produit à distribuer et au moins un mécanisme distributeur commandé par des moyens d'entraînement actionnés par des batteries, **caractérisé en ce qu'**il comporte deux réservoirs (7) et (10) pour les produits à distribuer, dans lequel un de ces réservoirs (7) incorpore une soupape (20) qui, à sa partie supérieure possède un bouton poussoir (19) qui est actionné par un élément pousseur (18) entraîné par le mouvement d'un moteur (15) qui est transmis au moyen des engrenages (16) et (17), ce moteur (15) étant actionné par ces batteries situées dans un bâti (22) et contrôlé par une plaque de contrôle (21), qui à son tour contrôle et combine le fonctionnement de ce moteur (15) avec celui d'un autre moteur (14) qui entraîne un ventilateur (13) qui fait circuler l'air qui est en contact avec un produit évaporé par un tuyau (11) situé dans l'autre de ces réservoirs (10), tous étant combinés simultanément et alternativement, conformément à la programmation de la plaque de contrôle (21).

2. - Distributeur plurivalent conformément à la revendication 1, **caractérisé en ce que** ce couvercle (5) comporte des grilles (24, 25, 26) à ses parties supérieure, centrale et inférieure, respectivement, qui font que le courant d'air généré par le ventilateur (13) circule à travers elles et l'air circulant entre en contact avec le produit évaporé du réservoir (10) par le tuyau (11).

3. - Distributeur plurivalent conformément à la revendication 2, **caractérisé en ce que** ce bouton poussoir (19) est un bouton poussoir pulvérisateur qui distribue le produit contenu dans le réservoir (7) directement à l'atmosphère extérieure à travers une fenêtre (27) qui se trouve à cet effet sur le couvercle avant (5) du distributeur, en combinaison simultanée ou alternative avec la distribution du produit contenu dans le réservoir (10) au moyen de ces grilles (24, 25 et 26).

4. Distributeur plurivalent conformément à la revendication 2, **caractérisé en ce que** ce bouton poussoir (19) distribue le produit contenu dans le réservoir (7) dans des petits tuyaux qui sont appuyés sur le châssis par une cheville ou un support (9) et traversés par la sortie (8) et dirigés vers les canaux ou tuyaux correspondants, pour être traités en gouttes en combinaison simultanée ou alternative avec la distribution du produit contenu dans le réservoir (10) au moyen de ces grilles (24, 25 et 26).
